(19) Europäisches Patentamt / European Patent Office / Office européen des brevets

(11) EP 3 212 674 B1

(12) EUROPEAN PATENT SPECIFICATION

(45) Date of publication and mention of the grant of the patent:
**22.08.2018 Bulletin 2018/34**

(21) Application number: **15791420.1**

(22) Date of filing: **21.10.2015**

(51) Int Cl.:
**C08B 13/00** (2006.01)    **C08L 1/32** (2006.01)
**A61K 47/38** (2006.01)    **C09D 101/32** (2006.01)

(86) International application number:
**PCT/US2015/056590**

(87) International publication number:
**WO 2016/069340 (06.05.2016 Gazette 2016/18)**

(54) **PROCESS FOR PREPARING AN ESTER OF A CELLULOSE ETHER**

VERFAHREN ZUR HERSTELLUNG EINES ESTERS AUS EINEM CELLULOSEETHER

PROCÉDÉ DE PRÉPARATION D'UN ESTER D'UN ÉTHER DE CELLULOSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.10.2014 US 201462073264 P**

(43) Date of publication of application:
**06.09.2017 Bulletin 2017/36**

(73) Proprietor: **Dow Global Technologies LLC Midland, MI 48674 (US)**

(72) Inventors:
• **PETERMANN, Oliver**
  **29699 Bomlitz (DE)**
• **SPREHE, Matthias**
  **29699 Bomlitz (DE)**

(74) Representative: **f & e patent
Braunsberger Feld 29
51429 Bergisch Gladbach (DE)**

(56) References cited:
WO-A1-2009/061815    WO-A1-2011/159626
WO-A1-2013/148154    WO-A1-2014/031448
WO-A1-2014/137779    WO-A1-2015/041973
US-A- 2 912 430    US-A- 3 497 496
US-A- 3 629 237

**Description**

FIELD

The present invention relates to an improved process for preparing an ester of a cellulose ether by reacting the cellulose ether with an aliphatic monocarboxylic acid anhydride and/or a dicarboxylic acid anhydride.

INTRODUCTION

Esters of cellulose ethers, their uses and processes for preparing them are generally known in the art. Various known esters of cellulose ethers are useful as enteric polymers for pharmaceutical dosage forms, such as methylcellulose phthalate, hydroxypropyl methylcellulose phthalate (HPMCP), methylcellulose succinate, or hydroxypropyl methylcellulose acetate succinate (HPMCAS). Enteric polymers are those that are resistant to dissolution in the acidic environment of the stomach. Dosage forms coated with such polymers protect the drug from inactivation or degradation in the acidic environment or prevent irritation of the stomach by the drug.

U.S. Patent No. 4,226,981 discloses a process for preparing mixed esters of cellulose ethers, such as HPMCAS, by esterifying hydroxypropyl methylcellulose with succinic anhydride and acetic anhydride in the presence of an alkali carboxylate as the esterification catalyst and acetic acid as the reaction medium.

US Patent No. 5,776,501 lists various cellulosic polymers, vinyl polymers or acrylic polymers as a basis for enteric coatings, such as cellulose acetate phthalate, cellulose acetate trimellitate, hydroxypropyl methylcellulose phthalate, hydroxypropyl methylcellulose acetate succinate and carboxymethylethyl cellulose, polyvinyl alcohol acetate phthalate, or copolymers of methacrylic acid and ethyl acrylate. More specifically, US Patent No. 5,776,501 teaches the usage of a water-soluble cellulose ether, such as hydroxypropyl methyl cellulose (HPMC), for producing hydroxypropyl methyl cellulose acetate maleate. The hydroxypropyl methyl cellulose acetate maleate is used as coating base for enteric pharmaceutical preparations. The water-soluble cellulose ether has a viscosity of 3 to 10 cp (= mPa·s), determined as a 2% by weight aqueous solution. If the viscosity is less than 3 cp, the finally obtained coating film for solid enteric pharmaceutical preparations is insufficient in strength, while if it exceeds 10 cp, the viscosity observed when the cellulose ether is dissolved in a solvent to carry out a substitution reaction becomes extremely high and homogeneous mixing and handling for further treatment is very difficult.

International patent applications WO 2005/115330 and WO 2011/159626 disclose the preparation of hydroxypropyl methylcellulose acetate succinate (HPMCAS). HPMC having an apparent viscosity of 2.4 to 3.6 cp is recommended as a starting material. Alternatively, a HPMC starting material of 600 to 60,000 Daltons, preferably 3000 to 50,000 Daltons, more preferably 6,000 to 30,000 Daltons is recommended. According to Keary [Keary, C.M.; Carbohydrate Polymers 45 (2001) 293-303, Tables 7 and 8] HPMC having a weight average molecular weight of about 85-100 kDa has a viscosity of about 50 mPa·s, determined as a 2% by weight aqueous solution. The produced HPMCAS is useful for enhancing the concentration of a dissolved active agent, for example for enhancing the bioavailability of poorly water-soluble drugs.

Unfortunately, the use of cellulose ethers having a viscosity in the range of 2.4 to 10 cp (= mPa·s) as starting materials for the esterification, as recommended in the prior art, requires depolymerization of cellulose ethers to a large degree prior to their esterification with an aliphatic monocarboxylic acid anhydride and/or a dicarboxylic acid anhydride. The known partial depolymerization of cellulose ethers with an acid, such as HCl, followed by neutralization before the esterification step results in a cost-intensive multistep production process. Processes for depolymerizing cellulose ethers are described, for example, in European Patent Applications EP 1,141,029; EP 210,917; and EP 1,423,433; and in US Patent No. 4,316,982.

U.S. Patent No. 3,629,237 discloses reacting phthalic anhydride with a cellulose ether in the presence of an organic acid and a catalyst, such as anhydrous sodium acetate and potassium acetate, and in the presence of alkali metal salts of oxyacids of halogens, such as potassium chlorate, potassium bromated, and sodium chlorate, which facilitates the production of low-viscosity acid phthalates of cellulose ethers. Hydroxypropyl methylcellulose (HPMC) having a viscosity of 15 to 100 cps, determined as a 2% by weight aqueous solution, is used as a starting material for the reaction with phthalic anhydride. However, partial depolymerization of cellulose ethers with an acid, such as HCl, followed by neutralization before the esterification step is still required when using the HPMCs having viscosities of 15 to 100 cps for the reaction with phthalic anhydride.

Accordingly, it would be desirable to provide a process for preparing an ester of a cellulose ether, wherein the cellulose ether does not have to be depolymerized to a substantial degree before it is esterified. It would particularly be desirable to provide a process for preparing an ester of a cellulose ether, wherein the cellulose ether does not have to be depolymerized at all before it is esterified.

SUMMARY

The present invention relates to a process for producing an esterified cellulose ether which comprises the step of reacting a cellulose ether with an aliphatic monocarboxylic acid anhydride and/or a dicarboxylic acid anhydride and with a depolymerizing agent, wherein the cellulose ether has an initial viscosity of at least 500 mPa·s, measured as a 2 weight-% solution in water at 20 °C, and the reaction of the cellulose ether with the aliphatic monocarboxylic acid anhydride and/or the dicarboxylic acid anhydride and with the depolymerizing agent is conducted simultaneously or in sequence in one reaction device.

DETAILED DESCRIPTION

The cellulose ether used as a starting material in the process of the present invention has an initial viscosity of at least 500 mPa·s, typically at least 700 mPa·s, preferably at least 1000 mPa·s more preferably at least 1500 mPa·s, even more preferably at least 2000 mPa·s and most preferably at least 3000 mPa·s, determined as a 2 weight-% solution in water at 20 °C. The cellulose ether generally has an initial viscosity of up to 100,000 mPa·s, typically up to 30,000 mPa·s, preferably up to 15,000 mPa·s, more preferably up to 7,000 mPa·s, and most preferably up to 5,000 mPa·s, determined as a 2 weight-% solution in water at 20 °C. The viscosity is determined as a 2 weight-% solution in water at 20 °C as described in the United States Pharmacopeia (USP 35, "Hypromellose", pages 423 - 424 and 3467 - 3469). As described in USP 35, viscosities of up to 600 mPa·s are determined by Ubbelohde viscosity measurement and viscosities above 600 mPa·s are determined using a Brookfield viscometer. Descriptions on preparing the 2 wt. % HPMC solution and both Ubbelohde and Brookfield viscosity measurement conditions are described USP 35. Cellulose ethers of such viscosity can be produced according to known procedures by alkalizing cellulose with an alkaline material and etherifying the produced alkali cellulose, without subjecting the cellulose, the alkali cellulose or the cellulose ether to a depolymerization reaction.

The term "initial viscosity" as used herein means the viscosity of the cellulose ether before partial depolymerization of the cellulose backbone by the depolymerizing agent. When cellulose ethers of such a high viscosity are used as starting material for the reaction with an aliphatic monocarboxylic acid anhydride and/or a dicarboxylic acid anhydride, a gel-like or dough-like inhomogeneous reaction mass results when the cellulose ether is combined with a diluent at a typical ratio of cellulose ether to diluent as described below. A homogeneous reaction mixture cannot be obtained by stirring the reaction mass. Large lumps of starting materials like cellulose ethers are formed which neither dissolve nor are properly dispersible in the diluent. It is very surprising that an effective esterification with an aliphatic monocarboxylic acid anhydride and/or a dicarboxylic acid anhydride and effective partial depolymerization with the depolymerizing agent can be achieved, without leaving a large amount of non-reacted or insufficiently reacted cellulose ether in spite of the physical properties of the reaction mass as described above.

A depolymerizing agent is used in the esterification process of the present invention. In a preferred aspect of the invention the depolymerizing agent is selected from oxidizing agents, typically oxidizing agents different from oxygen, such as ozone, peroxides, halites, halates, perhalates, hypohalites and perborates, and hydrogen peroxide. Preferred depolymerizing agents are alkali metal chlorites, alkali metal chlorates, such as potassium chlorate or sodium chlorate, alkali metal perchlorates, alkali metal periodates, alkali metal hypobromites, alkali metal hypochlorites, alkali metal hypoiodites, alkali metal peroxides, and hydrogen peroxide. Sodium and potassium are the preferred alkali metals.

In another aspect of the invention the depolymerizing agent is an inorganic acid, such as a hydrogen halide, preferably hydrogen chloride; sulfuric acid, nitric acid, phosphoric acid, or persulfuric acid. When using an inorganic acid as depolymerizing agent, a neutralizing agent, such as sodium hydroxide, sodium bicarbonate, or sodium carbonate, is typically added to the reaction mixture after the desired degree of depolymerization has been achieved.

The amount of depolymerizing agent is generally at least 0.005 mole, preferably at least 0.01 mole, more preferably at least 0.02 mole, and most preferably at least 0.03 mole, per mole of anhydroglucose units of the cellulose ether. The amount of depolymerizing agent is generally up to 1 mole, preferably up to 0.70 mole, more preferably up to 0.40 mole, and most preferably up to 0.30 mole, per mole of anhydroglucose units of the cellulose ether. One or more types of depolymerizing agents can be used, but their total amount is preferably chosen to be within the above described molar ratios between the depolymerizing agent and the anhydroglucose units of the cellulose ether.

The cellulose ether used as a starting material in the process of the present invention has a cellulose backbone having β-1,4 glycosidically bound D-glucopyranose repeating units, designated as anhydroglucose units in the context of this invention. The cellulose ether used as a starting material in the process of the present invention preferably is an alkyl cellulose, hydroxyalkyl cellulose or hydroxyalkyl alkylcellulose. This means that in the cellulose ether utilized in the process of the present invention, at least a part of the hydroxyl groups of the cellulose backbone of the anhydroglucose units are substituted by alkoxyl groups or hydroxyalkoxyl groups or a combination of alkoxyl and hydroxyalkoxyl groups. The hydroxyalkoxyl groups are typically hydroxymethoxyl, hydroxyethoxyl and/or hydroxypropoxyl groups. Hydroxyethoxyl and/or hydroxypropoxyl groups are preferred. Typically one or two kinds of hydroxyalkoxyl groups are present in the cellulose ether. Preferably a single kind of hydroxyalkoxyl group, more preferably hydroxypropoxyl, is present. The alkoxyl groups are typically methoxyl, ethoxyl and/or propoxyl groups. Methoxyl groups are preferred.

Illustrative of the above-defined cellulose ethers are alkylcelluloses, such as methylcellulose, ethylcellulose, and propylcellulose; hydroxyalkylcelluloses, such as hydroxyethylcellulose, hydroxypropylcellulose, and hydroxybutylcellulose; and hydroxyalkyl alkylcelluloses, such as hydroxyethyl methylcellulose, hydroxymethyl ethylcellulose, ethyl hydroxyethylcellulose, hydroxypropyl methylcellulose, hydroxypropyl ethylcellulose, hydroxybutyl methylcellulose, and hydroxybutyl ethylcellulose; and those having two or more hydroxyalkyl groups, such as hydroxyethylhydroxypropyl methylcellulose. Most preferably, the cellulose ether is a hydroxypropyl methylcellulose.

The degree of the substitution of hydroxyl groups of the anhydroglucose units by hydroxyalkoxyl groups is expressed by the molar substitution of hydroxyalkoxyl groups, the MS(hydroxyalkoxyl). The MS(hydroxyalkoxyl) is the average number of moles of hydroxyalkoxyl groups per anhydroglucose unit in the cellulose ether. It is to be understood that during the hydroxyalkylation reaction the hydroxyl group of a hydroxyalkoxyl group bound to the cellulose backbone can be further etherified by an alkylation agent, e.g. a methylation agent, and/or a hydroxyalkylation agent. Multiple subsequent hydroxyalkylation etherification reactions with respect to the same carbon atom position of an anhydroglucose unit yields a side chain, wherein multiple hydroxyalkoxyl groups are covalently bound to each other by ether bonds, each side chain as a whole forming a hydroxyalkoxyl substituent to the cellulose backbone.

The term "hydroxyalkoxyl groups" thus has to be interpreted in the context of the MS(hydroxyalkoxyl) as referring to the hydroxyalkoxyl groups as the constituting units of hydroxyalkoxyl substituents, which either comprise a single hydroxyalkoxyl group or a side chain as outlined above, wherein two or more hydroxyalkoxyl units are covalently bound to each other by ether bonding. Within this definition it is not important whether the terminal hydroxyl group of a hydroxyalkoxyl substituent is further alkylated or not; both alkylated and non-alkylated hydroxyalkoxyl substituents are included for the determination of MS(hydroxyalkoxyl). The esterified cellulose ether generally has a molar substitution of hydroxyalkoxyl groups of at least 0.05, preferably at least 0.08, more preferably at least 0.12, and most preferably at least 0.15. The degree of molar substitution is generally not more than 1.00, preferably not more than 0.90, more preferably not more than 0.70, and most preferably not more than 0.50.

The average number of hydroxyl groups substituted by alkoxyl groups, such as methoxyl groups, per anhydroglucose unit, is designated as the degree of substitution of alkoxyl groups, DS(alkoxyl). In the above-given definition of D S, the term "hydroxyl groups substituted by alkoxyl groups" is to be construed within the present invention to include not only alkylated hydroxyl groups directly bound to the carbon atoms of the cellulose backbone, but also alkylated hydroxyl groups of hydroxyalkoxyl substituents bound to the cellulose backbone. The cellulose ethers used as a starting material in the process of the present invention preferably have a DS(alkoxyl) of at least 1.0, more preferably at least 1.1, even more preferably at least 1.2, most preferably at least 1.4, and particularly at least 1.6. The DS(alkoxyl) is preferably not more than 2.5, more preferably not more than 2.4, even more preferably not more than 2.2, and most not more than 2.05. The degree of substitution of alkoxyl groups and the molar substitution of hydroxyalkoxyl groups can be determined by Zeisel cleavage of the cellulose ether with hydrogen iodide and subsequent quantitative gas chromatographic analysis (G. Bartelmus and R. Ketterer, Z. Anal. Chem., 286 (1977) 161-190). Most preferably the cellulose ether utilized in the process of the invention is hydroxypropyl methylcellulose having a DS(methoxyl) within the ranges indicated above for DS(alkoxyl) and an MS(hydroxypropoxyl) within the ranges indicated above for MS(hydroxyalkoxyl).

The cellulose ether is preferably used in its dried state as a starting material for producing the esterified cellulose ether. "Dried state" means that the cellulose ether has a water content of not more than 5 percent, based on the weight of the moist cellulose ether, as defined in European Pharmacopeia Ph Eur 6 2008, Hypromellose, page 2819. Typically the water content is not more than 3 percent, based on the weight of the moist cellulose ether. The cellulose ether, preferably an above-mentioned hydroxypropyl methylcellulose, is used as a starting material to produce an esterified cellulose ether, preferably hydroxypropyl methylcellulose acetate succinate (HPMCAS) as described below. The cellulose ether is reacted with an aliphatic monocarboxylic acid anhydride and/or a dicarboxylic acid anhydride and with a depolymerizing agent simultaneously or in sequence in one reaction device. Suitable reaction devices, such as batch reactors or reaction vessels, are known in the art. Preferred are reactors equipped with a stirring device or kneaders.

Preferred depolymerizing agents and preferred molar ratios between the depolymerizing agent and the anhydroglucose units of the cellulose ether are as described further above. Esterification of the cellulose ether with the aliphatic monocarboxylic acid anhydride and/or the dicarboxylic acid anhydride can be partially or fully completed before the cellulose ether carrying additional ester groups is reacted with the depolymerizing agent. Alternatively, the cellulose ether can be first reacted with the depolymerizing agent and then with the aliphatic monocarboxylic acid anhydride and/or the dicarboxylic acid anhydride. Even if the cellulose ether is reacted with the aliphatic monocarboxylic acid anhydride and/or the dicarboxylic acid anhydride and with a depolymerizing agent in sequence, the reactions take place in the same reaction device. Intermediate isolation steps or purification steps are not necessary and typically not carried out. Preferably, the reaction of the cellulose ether with an aliphatic monocarboxylic acid anhydride and/or a dicarboxylic acid anhydride and with a depolymerizing agent is conducted simultaneously. More preferably a reaction mixture is prepared which comprises the cellulose ether, the aliphatic monocarboxylic acid anhydride and/or the dicarboxylic acid anhydride and the depolymerizing agent and the reaction mixture is subsequently heated to conduct the esterification reaction.

Preferred aliphatic monocarboxylic acid anhydrides are selected from the group consisting of acetic anhydride, butyric anhydride and propionic anhydride. Preferred dicarboxylic acid anhydrides are selected from the group consisting of succinic anhydride, maleic anhydride and phthalic anhydride. A preferred aliphatic monocarboxylic acid anhydride can be used alone; or a preferred dicarboxylic acid anhydride can be used alone; or a preferred aliphatic monocarboxylic acid anhydride can be used in combination with a preferred dicarboxylic acid anhydride. If an aliphatic monocarboxylic acid anhydride and a dicarboxylic acid anhydride are used for esterifying the cellulose ether, the two anhydrides may be introduced into the reaction device at the same time or separately one after the other.

The amount of each anhydride to be introduced into the reaction device is determined depending on the desired degree of esterification to be obtained in the final product, usually being 1 to 10 times the stoichiometric amounts of the desired molar degree of substitution of the anhydroglucose units by esterification. The molar ratio between the anhydride of an aliphatic monocarboxylic acid and the anhydroglucose units of the cellulose ether generally is 0.1 / 1 or more, preferably 0.3 / 1 or more, more preferably 0.5 / 1 or more, most preferably 1 / 1 or more, and particularly 1.5 / 1 or more. The molar ratio between the anhydride of an aliphatic monocarboxylic acid and the anhydroglucose units of the cellulose ether generally is 17 / 1 or less, preferably 10 / 1 or less, more preferably 8 / 1 or less, most preferably 6 / 1 or less, and particularly 4 / 1 or less. The molar ratio between the anhydride of a dicarboxylic acid and the anhydroglucose units of cellulose ether preferably is 0.01 / 1 or more, more preferably 0.04 / 1 or more, and most preferably 0.2 / 1 or more. The molar ratio between the anhydride of a dicarboxylic acid and the anhydroglucose units of cellulose ether preferably is 2.5 / 1 or less, more preferably 1.5 / 1 or less, and most preferably 1 / 1 or less.

The molar number of anhydroglucose units of the cellulose ether utilized in the process of the present invention can be determined from the weight of the cellulose ether used as a starting material, by calculating the average molecular weight of the substituted anhydroglucose units from the DS(alkoxyl) and MS(hydroxyalkoxyl).

The esterification of the cellulose ether is typically conducted in a diluent, preferably in an aliphatic carboxylic acid as a reaction medium, such as acetic acid, propionic acid, or butyric acid. The reaction medium can comprise minor amounts of other solvents or diluents which are liquid at room temperature and do not react with the cellulose ether, such as aromatic or aliphatic solvents like benzene, toluene, 1,4-dioxane, or tetrahydrofurane; or halogenated $C_1$-$C_3$ derivatives, like dichloro methane or dichloro methyl ether, but the amount of the aliphatic carboxylic acid should generally be more than 50 percent, preferably at least 75 percent, and more preferably at least 90 percent, based on the total weight of the reaction medium. Most preferably the reaction medium consists of an aliphatic carboxylic acid. The esterification reaction is generally conducted in the presence of at least 200 parts, preferably at least 300 parts, more preferably at least 400 parts, and most preferably at least 600 parts by weight of the reaction medium, such as an aliphatic carboxylic acid, per 100 weight parts of the cellulose ether. The esterification reaction is generally conducted in the presence of up to 2000 parts, preferably up to 1500 parts, more preferably up to 1000 parts and most preferably up to 600 parts by weight of the reaction medium, such as an aliphatic carboxylic acid, per 100 weight parts of the cellulose ether.

The esterification reaction is generally conducted in the presence of an esterification catalyst, preferably in the presence of an alkali metal carboxylate, such as sodium acetate or potassium acetate. The amount of the alkali metal carboxylate is generally 20 to 200 parts by weight of the alkali metal carboxylate per 100 parts by weight of the cellulose ether. The molar ratio [alkali metal carboxylate / anhydroglucose units of cellulose ether] is preferably from [0.4 / 1.0] to [3.8 / 1.0], more preferably from [1.5 / 1.0] to [3.5/ 1.0], and most preferably from [1.9 / 1.0] to [3.0/ 1.0].

The reaction mixture is generally heated at 60 °C to 110 °C, preferably at 70 to 100 °C, for a period of time sufficient to complete the reaction, that is, typically from 2 to 25 hours, more typically from 2 to 8 hours.

The cellulose ether having an initial viscosity of at least 500 mPa·s, typically at least 700 mPa·s, preferably at least 1000 mPa·s more preferably at least 1500 mPa·s, even more preferably at least 2000 mPa·s and most preferably at least 3000 mPa·s, measured as a 2 weight-% solution in water at 20 °C, which is used as starting material in the process of the present invention has a very high thickening effect. As stated above, the viscosity is determined as a 2 weight-% solution in water at 20 °C as described in the United States Pharmacopeia (USP 35, "Hypromellose", pages 423 - 424 and 3467 - 3469).

When the cellulose ether is combined with the aliphatic carboxylic acid at a weight ratio as disclosed above and with the other reactants as described above, a gel-like or dough-like inhomogeneous reaction mass results. A homogeneous reaction mixture cannot be obtained by stirring the reaction mass. Large lumps of starting materials like cellulose ethers are formed which neither dissolve nor are properly dispersible in the aliphatic carboxylic acid. Surprisingly, the inhomogeneous reaction mixture does not affect the quality of the produced esterified cellulose ether and the esterification reaction proceeds well. While the esterification and the partial depolymerization proceed, the viscosity of the reaction mixture decreases and its stirrability improves, but a homogeneous mixture generally can only be achieved after an extended time period, typically after 1 to 3 hours.

After completion of the esterification reaction, the reaction product can be precipitated from the reaction mixture in a known manner, for example by contacting the reaction mixture with a large volume of water, such as described in U.S. Patent No. 4,226,981, International Patent Application WO 2005/115330 or European Patent Application EP 0 219 426. In a preferred embodiment of the invention the reaction product is precipitated from the reaction mixture as described in International Patent Application PCT/US13/030394, published as WO2013/148154, to produce an esterified cellulose ether in the form of a powder.

According to the process of the present invention an esterified cellulose ether is produced that has (i) aliphatic monovalent acyl groups and/or (ii) groups of the formula - C(O) - R - COOA, wherein R is a divalent aliphatic or aromatic hydrocarbon group and A is hydrogen or a cation. The cation preferably is an ammonium cation, such as $NH_4^+$ or an alkali metal ion, such as the sodium or potassium ion, more preferably the sodium ion. Most preferably, A is hydrogen. The aliphatic monovalent acyl groups are preferably selected from the group consisting of acetyl, propionyl, and butyryl, such as n-butyryl or i-butyryl. Preferred groups of the formula -C(O)-R-COOA are -C(O)-$CH_2$-$CH_2$-COOA, such as -C(O)-$CH_2$-$CH_2$-COOH or -C(O)-$CH_2$-$CH_2$-COO$^-$Na$^+$, -C(O)-CH=CH-COOA, such as -C(O)-CH=CH-COOH or -C(O)-CH=CH-COO$^-$Na$^+$, or -C(O)-$C_6H_4$-COOA, such as -C(O)-$C_6H_4$-COOH or -C(O)-$C_6H_4$-COO$^-$Na$^+$. In the groups of formula -C(O)-$C_6H_4$-COOA the carbonyl group and the carboxylic group are preferably arranged in ortho-positions.

Preferred esterified cellulose ethers produced according to the process of the present invention are:

i) HPMCXY and HPMCX, wherein HPMC is hydroxypropyl methyl cellulose, X is A (acetate), or X is B (butyrate) or X is Pr (propionate) and Y is S (succinate), or Y is P (phthalate) or Y is M (maleate), such as hydroxypropyl methyl cellulose acetate phthalate (HPMCAP), hydroxypropyl methyl cellulose acetate maleate (HPMCAM), hydroxypropyl methylcellulose acetate succinate (HPMCAS) or hydroxypropyl methyl cellulose acetate (HPMCA); or

ii) hydroxypropyl methyl cellulose phthalate (HPMCP); hydroxypropyl cellulose acetate succinate (HPCAS), hydroxybutyl methyl cellulose propionate succinate (HBMCPrS), hydroxyethyl hydroxypropyl cellulose propionate succinate (HEHPCPrS); and methyl cellulose acetate succinate (MCAS).

Hydroxypropyl methylcellulose acetate succinate (HPMCAS) is the most preferred esterified cellulose ether.

The esterified cellulose ethers produced according to the process of the present invention preferably have a DS(methoxyl) and an MS(hydroxyalkoxyl) as indicated further above. The esterified cellulose ethers produced according to the process of the present invention have a degree of substitution of aliphatic monovalent acyl groups, such as acetyl, propionyl, or butyryl groups, of 0 (zero) or preferably at least 0.05, more preferably at least 0.10, most preferably at least 0.15, and particularly at least 0.20. The degree of substitution of aliphatic monovalent acyl groups is generally up to 1.75, preferably up to 1.50, more preferably up to 1.25, and most preferably up to 1.00. The esterified cellulose ethers generally have a degree of substitution of groups of formula -C(O) - R - COOA, such as succinoyl, of 0 (zero) or preferably at least 0.05, more preferably at least 0.10. The degree of substitution of groups of formula -C(O) - R - COOA, such as succinoyl, is generally up to 1.6, preferably up to 1.30, more preferably up to 1.00, most preferably up to 0.70, and particularly up to 0.60. The sum of i) the degree of substitution of aliphatic monovalent acyl groups and ii) the degree of substitution of groups of formula -C(O) - R - COOA is greater than 0. It is generally at least 0.10, preferably at least 0.20, more preferably at least 0.30, and most preferably at least 0.40. This sum is generally up to 1.9, preferably up to 1.55, more preferably up to 1.15, and particularly up to 1.00.

The content of the acetate and succinate ester groups is determined according to "Hypromellose Acetate Succinate, United States Pharmacopia and National Formulary, NF 29, pp. 1548-1550". Reported values are corrected for volatiles (determined as described in section "loss on drying" in the above HPMCAS monograph).The method may be used in analogue manner to determine the content of propionyl, butyryl, phthalyl and other ester groups.

The content of ether groups in the esterified cellulose ether is determined in the same manner as described for "Hypromellose", United States Pharmacopeia and National Formulary, USP 35, pp 3467-3469.

The contents of ether and ester groups obtained by the above analyses are converted to DS and MS values of individual substituents according to the formulas below. The formulas may be used in analogue manner to determine the DS and MS of substituents of other cellulose ether esters.

$$\% \text{ cellulose backbone}$$
$$= 100 - \left( \%\text{MeO} * \frac{\text{M(OCH}_3) - \text{M(OH)}}{\text{M(OCH}_3)} \right)$$
$$- \left( \%\text{HPO} * \frac{\text{M(OCH}_2\text{CH(OH)CH}_3) - \text{M(OH)}}{\text{M(OCH}_2\text{CH(OH)CH}_3)} \right)$$
$$- \left( \%\text{Acetyl} * \frac{\text{M(COCH}_3) - \text{M(H)}}{\text{M(COCH}_3)} \right)$$
$$- \left( \%\text{Succinoyl} * \frac{\text{M(COC}_2\text{H}_4\text{COOH}) - \text{M(H)}}{\text{M(COC}_2\text{H}_4\text{COOH})} \right)$$

$$DS(Me) = \frac{\dfrac{\%MeO}{M(OCH_3)}}{\dfrac{\%cellulose\ backbone}{M(AGU)}} \qquad MS(HP) = \frac{\dfrac{\%HPO}{M(HPO)}}{\dfrac{\%cellulose\ backbone}{M(AGU)}}$$

$$DS(Acetyl) = \frac{\dfrac{\%Acetyl}{M(Acetyl)}}{\dfrac{\%cellulose\ backbone}{M(AGU)}} \qquad DS(Succinoyl) = \frac{\dfrac{\%Succinoyl}{M(Succinoyl)}}{\dfrac{\%cellulose\ backbone}{M(AGU)}}$$

$M(MeO) = M(OCH_3) = 31.03$ Da $M(HPO) = M(OCH_2CH(OH)CH_3) = 75.09$ Da $M(Acetyl) = M(COCH_3) = 43.04$ Da $M(Succinoyl) = M(COC_2H_4COOH) = 101.08$ Da $M(AGU) = 162.14$ Da $M(OH) = 17.008$ Da $M(H) = 1.008$ Da

By convention, the weight percent is an average weight percentage based on the total weight of the cellulose repeat unit, including all substituents. The content of the methoxyl group is reported based on the mass of the methoxyl group (i.e., -$OCH_3$). The content of the hydroxyalkoxyl group is reported based on the mass of the hydroxyalkoxyl group (i.e., -O-alkylene-OH); such as hydroxypropoxyl (i.e., -O-$CH_2$CH($CH_3$)-OH). The content of acetyl group is reported based on the mass of - C(O)-$CH_3$. The content of the succinoyl groups is reported based on the mass of this group, i.e., -C(O)-$CH_2$-$CH_2$-COOH.

According to the above described process esterified cellulose ethers are produced which generally have a weight average molecular weight $M_w$ of 10,000 Dalton or more, preferably 20,000 Dalton or more, more preferably 30,000 Dalton or more, and most preferably 80,000 Dalton or more. The esterified cellulose ethers generally have a weight average molecular weight $M_w$ of up to 500,000 Dalton, preferably up to 450,000 Dalton, more preferably up to 350,000 Dalton, even more preferably up to 250,000 Dalton, and particularly up to 200,000 Dalton or up to 120,000 Dalton. The weight average molecular weight $M_w$ of the esterified cellulose ether is typically at least 25 percent lower, more typically at least 40 percent lower than the weight average molecular weight of the cellulose ether used as a starting material for producing the esterified cellulose ether, which reduction in $M_w$ is caused by the treatment with the depolymerizing agent.

The esterified cellulose ethers typically have a Polydispersity $M_w/M_n$ of at least 1.3, and more typically at least 1.5. Moreover, the esterified cellulose ethers typically have a Polydispersity of up to 3.5, preferably up to 3.0, more preferably up to 2.8, and most preferably of up to 2.6. The Polydispersity $M_w/M_n$ is calculated based on the determination of the weight average molecular weight $M_w$ and the number average molecular weight $M_n$.

The esterified cellulose ethers generally have a number average molecular weight $M_n$ of 5000 Dalton or more, preferably 10,000 Dalton or more, more preferably 20,000 Dalton or more, and most preferably 25,000 Dalton or more. The esterified cellulose ethers generally have a number average molecular weight $M_n$ of up to 150,000 Dalton, preferably up to 110,000 Dalton, more preferably up to 90,000 Dalton, and most preferably up to 50,000 Dalton.

$M_w$ and $M_n$ are measured by SEC-MALLS using as mobile phase a mixture which has been produced by mixing 40 parts by volume of acetonitrile and 60 parts by volume of aqueous buffer containing 50 mM $NaH_2PO_4$ and 0.1 M $NaNO_3$. The mobile phase is adjusted to a pH of 8.0. SEC-MALLS stands for Size Exclusion Chromatography coupled with a mass sensitive Multi Angle Laser Light Scattering detector. The procedure is described in Journal of Pharmaceutical and Biomedical Analysis 56 (2011) 743-748. The measurement of $M_w$ and $M_n$ is described in more details in the Examples.

The esterified cellulose ethers generally have a viscosity of up to 200 mPa·s, preferably up to 100 mPa·s, more preferably up to 50 mPa·s, even more preferably up to 30 mPa·s, most preferably up to 10 mPa·s, and particularly up to 5 mPa·s, measured as a 2.0 wt.-% solution of the esterified cellulose ether in 0.43 wt.-% aqueous NaOH at 20 °C. Generally the viscosity is at least 1.2 mPa·s, typically at least 1.8 mPa·s, and more typically at least 2.4 mPa·s, measured as a 2.0 wt.-% solution of the esterified cellulose ether in 0.43 wt.-% aqueous NaOH at 20 °C. The 2.0 % by weight solution of the esterified cellulose ether is prepared as described in"Hypromellose Acetate Succinate, United States Pharmacopia and National Formulary, NF 29, pp. 1548-1550", followed by an Ubbelohde viscosity measurement according to DIN 51562-1:1999-01 (January 1999).

Some embodiments of the invention will now be described in detail in the following Examples.

EXAMPLES

Unless otherwise mentioned, all parts and percentages are by weight. In the Examples the following test procedures are used.

Viscosity of Hydroxypropyl Methyl Cellulose (HPMC) samples

The viscosity of the cellulose ether was determined as a 2 weight-% solution in water at 20 °C as described in the United States Pharmacopeia (USP 35, "Hypromellose", pages 423 - 424 and 3467 - 3469). As described in the United States Pharmacopeia, viscosities of less than 600 mPa·s were determined by an Ubbelohde viscosity measurement and viscosities of 600 mPa·s or more were determined using a Brookfield viscometer. Descriptions on preparing the 2 wt. % HPMC solution and both Ubbelohde and Brookfield viscosity measurement conditions are disclosed in the United States Pharmacopeia (USP 35, "Hypromellose", pages 423 - 424 and 3467 - 3469 and in ASTM D-445 and ISO 3105 referenced therein).

Viscosity of Hydroxypropyl Methyl Cellulose Acetate Succinate (HPMCAS)

The 2.0 % by weight solution of the HPMCAS in 0.43 wt.-% aqueous NaOH was prepared as described in"Hypromellose Acetate Succinate, United States Pharmacopia and National Formulary, NF 29, pp. 1548-1550", followed by an Ubbelohde viscosity measurement at 20 °C according to DIN 51562-1:1999-01 (January 1999).

Content of ether and ester groups of HPMCAS

The content of ether groups in the HPMCAS was determined in the same manner as described for "Hypromellose", United States Pharmacopeia and National Formulary, USP 35, pp 3467-3469.

The ester substitution with acetyl groups ($-CO-CH_3$) and the ester substitution with succinoyl groups ($-CO-CH_2-CH_2-COOH$) were determined according to Hypromellose Acetate Succinate, United States Pharmacopia and National Formulary, NF 29, pp. 1548-1550". Reported values for ester substitution were corrected for volatiles (determined as described in section "loss on drying" in the above HPMCAS monograph).

Determination of $M_w$ and $M_n$ of HPMCAS

Mw and Mn were measured according to Journal of Pharmaceutical and Biomedical Analysis 56 (2011) 743 unless stated otherwise. The mobile phase was a mixture of 40 parts by volume of acetonitrile and 60 parts by volume of aqueous buffer containing 50 mM $NaH_2PO_4$ and 0.1 M $NaNO_3$. The mobile phase was adjusted to a pH of 8.0. Solutions of the cellulose ether esters were filtered into a HPLC vial through a syringe filter of 0.45 $\mu$m pore size.

More specifically, the utilized Chemicals and solvents were:
Polyethylene oxide standard materials (abbreviated as PEOX 20 K and PEOX 30 K) were purchased from Agilent Technologies, Inc. Palo Alto, CA, catalog number PL2083-1005 and PL2083-2005.

Acetonitrile (HPLC grade ≥ 99.9 %, CHROMASOL plus), catalog number 34998, sodium hydroxide (semiconductor grade, 99.99 %, trace metal base), catalog number 306576, water (HPLC grade, CHROMASOLV Plus) catalog number 34877 and sodium nitrate (99,995 %, trace metal base) catalog number 229938 were purchased from Sigma-Aldrich, Switzerland.

Sodium dihydrogen phosphate (≥ 99.999 % TraceSelect) catalog number 71492 was purchased from FLUKA, Switzerland.

The normalization solution of PEOX20 K at 5 mg/mL, the standard solution of PEOX30 K at 2 mg/mL, and the sample solution of HPMCAS at 2 mg/mL were prepared by adding a weighed amount of polymer into a vial and dissolving it with a measured volume of mobile phase. All solutions were allowed to dissolve at room temperature in the capped vial for 24 h with stirring using a PTFE-coated magnetic stirring bar.

The normalization solution (PEOX 20k, single preparation, N) and the standard solution (PEOX30 K, double preparation, S1 and S2) were filtered into a HPLC vial through a syringe filter of 0.02 $\mu$m pore size and 25 mm diameter (Whatman Anatop 25, catalog number 6809-2002), Whatman.

The test sample solution (HPMCAS, prepared in duplicate, T1, T2) and a laboratory standard (HPMCAS, single preparation, LS) were filtered into a HPLC vial through a syringe filter of 0.45 $\mu$m pore size (Nylon, e.g. Acrodisc 13 mm VWR catalog number 514-4010).

Chromatographic condition and run sequence were conducted as described by Chen, R. et al.; Journal of Pharmaceutical and Biomedical Analysis 56 (2011) 743- 748). The SEC-MALLS instrument set-up included a HP1100 HPLC system from Agilent Technologies, Inc. Palo Alto, CA; a DAWN Heleos II 18 angle laser light scattering detector and a OPTILAB rex refractive index detector, both from Wyatt Technologies, Inc. Santa Barbara, CA. The analytical size exclusion column (TSK-GEL® GMPWXL, 300 × 7.8 mm) was purchased from Tosoh Bioscience. Both the OPTILAB and the DAWN were operated at 35 °C. The analytical SEC column was operated at room temperature (24 ± 5 °C). The mobile phase was a mixture of 40 volume parts of acetonitrile and 60 volume parts of aqueous buffer containing 50 mM NaH2PO4 and 0.1 M NaNO3 prepared as follows:

Aqueous buffer: 7.20 g of sodium dihydrogen phosphate and 10.2 g of sodium nitrate were added to 1.2 L purified water in a clean 2 L glass bottle under stirring until dissolution.

Mobile phase: 800 mL of acetonitrile were added to 1.2 L of the aqueous buffer prepared above, and stirred until a good mixture was achieved and the temperature equilibrated to ambient temperature. The mobile phase was pH adjusted to 8.0 with 10M NaOH and filtered through a 0.2 m nylon membrane filter. The flow rate was 0.5 mL/min with in-line degassing. The injection volume was 100 $\mu$L and the analysis time was 35 min.

The MALLS data were collected and processed by Wyatt ASTRA software (version 5.3.4.20) using dn/dc value (refractive index increment) of 0.120 mL/g for HPMCAS. The light scattering signals of detector Nos. 1-4, 17, and 18) were not used in the molecular weight calculation. A representative chromatographic run sequence is given below: B, N, LS, S1 (5x), S2, T1 (2x), T2 (2x), T3 (2x), T4 (2x), S2, T5(2x), etc., S2, LS, W, where, B represents blank injection of mobile phase, N1 represents normalization solution; LS represents a laboratory standard HPMCAS; S1 and S2 represent standard solutions one and two, respectively; T1, T2, T3, T4, and T5 represent test sample solutions and W represents water injection. (2x) and (5x) denote the number of injections of the same solution.

Both the OPTILAB and the DAWN were calibrated periodically according to the manufacturer's recommended procedures and frequency. A 100 $\mu$L injection of a 5 mg/mL polyethylene oxide standard (PEOX20 K) was employed for normalizing all angle light scattering detectors relative to 90° detector for each run sequence.

Use of this mono-dispersed polymer standard also enabled the volume delay between the OPTILAB and the DAWN to be determined, permitting proper alignment of the light scattering signals to the refractive index signal. This is necessary for the calculation of the weight-averaged molecular weight (Mw) for each data slice.

Utilized Hydroxypropyl methylcellulose (HPMC)

The HPMC E3 LV had a methoxyl substitution ($DS_M$) and hydroxypropoxyl substitution ($MS_{HP}$) as listed in Table 2 below and a viscosity of 3.2 mPa·s, measured as a 2 wt.-% solution in water at 20 °C according to the Ubbelohde method described above. The weight average molecular weight of the HPMC E3 LV was about 20,000 Dalton. The HPMC E3 LV is a low viscosity HPMC which is commercially available from The Dow Chemical Company as Methocel E3 LV Premium cellulose ether. It is produced by partial depolymerization of higher molecular weight HPMC.

The HPMC E4M had a methoxyl substitution ($DS_M$) and hydroxypropoxyl substitution ($MS_{HP}$) as listed in Table 2 below and a viscosity of 3729 mPa·s, measured as a 2 % solution in water at 20 °C according to the Brookfield method described above using Rotor No. 4 at 60 rpm. The weight average molecular weight of the HPMC E4M was 305330 Dalton (305 kDa). The HPMC E4M is a high viscosity HPMC which is commercially available from The Dow Chemical Company as Methocel E4M cellulose ether.

Production of HPMCAS of Examples 1 - 8

Glacial acetic acid, HPMC E4M, acetic anhydride, succinic anhydride, sodium acetate (water free) and potassium chlorate were introduced in the amounts listed in Table 1 below into a reaction vessel of 3L volume. The glacial acetic acid was readily absorbed by the HPMC powder. The mass in the reaction vessel had a solid appearance. The mass in the reaction vessel was heated at 85° C for 3.5 hours to effect esterification. During the heating period the mass formed a large lump and could not be stirred. At 85° C the mass could be stirred but no homogeneous mixture could be achieved. While the esterification and the partial depolymerization proceeded, the stirring ability of reaction mixture improved, but a homogeneous mixture could still not be achieved for an extended time period. After the reaction had proceeded for 1 hour, the reaction mass still displayed inhomogeneous lumps. After the completion of the reaction after 3.5 hours a homogeneous reaction solution was formed as during the reaction in Comparative Example A. Then 1.8 L of cold water was added to the reaction mixture under stirring to precipitate the HPMCAS. The precipitated product was removed from the reactor and washed with 20 L of water by applying high shear mixing using an Ultra-Turrax stirrer S50-G45 running at 5200 rpm. The product was isolated by filtration and dried at 55°C overnight.

In Example 3 the amount of acetic acid was increased to evaluate whether the stirrability of the reaction mass would be improved by an increased amount of acetic acid. However, no significant improvement was detectable.

Production of HPMCAS of Comparative Example A

The procedure of Examples 1 - 8 was repeated, except that low viscosity HPMC E3 LV was used instead of high viscosity HPMC E4M and the reaction was carried out in the absence of potassium chlorate. The reaction mixture consisting of glacial acetic acid, HPMC E3 LV, acetic anhydride, succinic anhydride and sodium acetate (water free) as listed in Table 1 below was well stirrable from the start.

The properties of the produced HPMCAS are listed in Table 2 below. All HPMCAS products of Examples 1 - 8 were white to off-white powders. The comparison of the HPMCAS produced according to Examples 1 - 8 and Comparative Example A illustrates the surprising fact that essentially the same degree of esterification is achieved using the same amount of esterification agents regardless whether a low viscosity cellulose ether is used as a starting material as in Comparative Example A or a high viscosity cellulose ether as in Examples 1 - 8. Also, essentially the same or an even lower polydispersity, $M_w/M_n$, is achieved in Examples 1 - 4 as in Comparative Example A. The $M_w$ and $M_n$ of the HPMCAS of Comparative Example A are within the ranges of $M_w$ and $M_n$ for Examples 1 - 4. The viscosity, determined as a 2.0 wt.-% solution in 0.43 wt. % aqueous NaOH, of the HPMCAS of Comparative Example A is even slightly higher than the viscosity of the HPMCAS of Examples 1 - 4. Essentially the same or an even lower polydispersity in Examples 1 - 4 means a comparable or even a more homogeneous distribution of the lengths of the polymer chains in Examples 1 - 4 than in Comparative Example A. This is highly surprising considering that no homogeneous reaction mixture could be achieved in Examples 1 - 8 by stirring, as explained in greater details above. The present invention avoids the known partial depolymerization of cellulose ethers with an acid, such as HCl, followed by neutralization before the esterification step. Hence the cost-intensive multistep production process of the prior art is simplified and the number of reaction steps is reduced. Thus the production costs are significantly reduced while the same quality of esterified cellulose ether is achieved. Moreover, controlling and varying the amount of depolymerizing agent utilized in the process of the present invention is a simple means for controlling and varying the number average molecular weight $M_n$ and the weight average molecular weight $M_w$ of the produced esterified cellulose ether.

Table 1

| (Comp.) Example | Type HPMC used as starting material | Amount HPMC* | | acetic acid | | Succinic anhydride | | Acetic anhydride | | Sodium acetate | | Potassium chlorate | |
|---|---|---|---|---|---|---|---|---|---|---|---|---|---|
| | | g | mol | g | mol/mol HPMC | g | mol/mol HPMC | g | mol/mol HPMC | g | mol/mol HPMC | g | mol/mol HPMC |
| A | HPMC E3 LV | 150 | 0.74 | 750 | 16.8 | 42.0 | 0.57 | 195.0 | 2.69 | 150.0 | 2.47 | -- | -- |
| 1 | HPMC E4M | 150 | 0.74 | 750 | 16.8 | 42.0 | 0.57 | 195.0 | 2.69 | 150.0 | 2.47 | 20 | 0.22 |
| 2 | HPMC E4M | 150 | 0.74 | 750 | 16.8 | 42.0 | 0.57 | 195.0 | 2.69 | 150.0 | 2.47 | 15 | 0.165 |
| 3 | HPMC E4M | 150 | 0.74 | 750 + 320 | | 42.0 | 0.57 | 195.0 | 2.69 | 150.0 | 2.47 | 10 | 0.11 |
| 4 | HPMC E4M | 150 | 0.74 | 750 | 16.8 | 42.0 | 0.57 | 195.0 | 2.69 | 150.0 | 2.47 | 7.5 | 0.083 |
| 5 | HPMC E4M | 150 | 0.74 | 750 | 16.8 | 42.0 | 0.57 | 195.0 | 2.69 | 150.0 | 2.47 | 4 | 0.044 |
| 6 | HPMC E4M | 150 | 0.74 | 750 | 16.8 | 42.0 | 0.57 | 195.0 | 2.69 | 150.0 | 2.47 | 3 | 0.033 |
| 7 | HPMC E4M | 150 | 0.74 | 750 | 16.8 | 42.0 | 0.57 | 195.0 | 2.69 | 150.0 | 2.47 | 2 | 0.022 |
| 8 | HPMC E4M | 150 | 0.74 | 750 | 16.8 | 42.0 | 0.57 | 195.0 | 2.69 | 150.0 | 2.47 | 1 | 0.011 |
| * calculated on the dried basis | | | | | | | | | | | | | |

Table 2

| (Comp.) Example | Molecular weight (kDa) | | | Ether Substitution | | Ester substitution | | Ether Substitution | | Ester substitution | | 2% viscosity in NaOH [mPa·s] |
|---|---|---|---|---|---|---|---|---|---|---|---|---|
| | Mn | Mw | Mw/$M_n$ | Methoxyl (%) | Hydroxypropoxyl (%) | Acetyl (%) | Succinoyl (%) | $DS_M$ | $MS_{HP}$ | $DOS_{AC}$ | $DOS_s$ | |
| A | 23 | 44 | 1.9 | 23.6 | 7.7 | 8.8 | 11.8 | 1.95 | 0.26 | 0.53 | 0.30 | 2.93 |
| 1 | 15 | 27 | 1.8 | 21.5 | 7.2 | 10.0 | 11.9 | 1.78 | 0.25 | 0.60 | 0.30 | 1.66 |
| 2 | 17 | 31 | 1.8 | 21.9 | 7.3 | 9.3 | 11.6 | 1.79 | 0.25 | 0.55 | 0.29 | 1.79 |
| 3 | 24 | 37 | 1.5 | 22.5 | 7.6 | 8.0 | 11.1 | 1.80 | 0.25 | 0.46 | 0.27 | 2.37 |
| 4 | 29 | 53 | 1.8 | 22.2 | 7.4 | 8.9 | 11.9 | 1.82 | 0.25 | 0.53 | 0.30 | 2.39 |
| 5 | 37 | 81 | 2.2 | 23.0 | 7.5 | 8.7 | 11.6 | 1.88 | 0.25 | 0.51 | 0.29 | 3.52 |
| 6 | 43 | 101 | 2.3 | 23.0 | 7.5 | 8.9 | 11.5 | 1.89 | 0.25 | 0.53 | 0.29 | 4.38 |
| 7 | 50 | 138 | 2.8 | 23.2 | 7.5 | 8.7 | 11.5 | 1.90 | 0.25 | 0.51 | 0.29 | 5.68 |
| 8 | 83 | 253 | 3.0 | 23.1 | 7.6 | 8.7 | 11.7 | 1.90 | 0.26 | 0.52 | 0.30 | 10.2 |

**Claims**

1. A process for producing an esterified cellulose ether comprising the step of reacting a cellulose ether with an aliphatic monocarboxylic acid anhydride and/or a dicarboxylic acid anhydride in the presence of up to 2000 weight parts of an aliphatic carboxylic acid per 100 weight parts of cellulose ether and with a depolymerizing agent, wherein the cellulose ether has an initial viscosity of at least 500 mPa·s, measured as a 2 weight-% solution in water at 20 °C, the depolymerizing agent is an inorganic acid or an oxidizing agent different from oxygen and the reaction of the cellulose ether with the aliphatic monocarboxylic acid anhydride and/or the dicarboxylic acid anhydride and with the depolymerizing agent is conducted simultaneously or in sequence in one reaction device.

2. The process of claim 1 wherein the cellulose ether has a viscosity of at least 1000 mPa·s, measured as a 2 weight-% solution in water at 20 °C.

3. The process of claim 1 or 2 wherein a reaction mixture comprising the cellulose ether, the aliphatic monocarboxylic acid anhydride and/or the dicarboxylic acid anhydride and the depolymerizing agent is prepared and the reaction mixture is subsequently heated to conduct the esterification reaction.

4. The process of any one of claims 1 to 3 wherein the amount of the depolymerizing agent is at least 0.03 mole per mole of anhydroglucose units of the cellulose ether.

5. The process of any one of claims 1 to 4 wherein the depolymerizing agent is selected from the group consisting of alkali metal chlorites, alkali metal chlorates, alkali metal perchlorates, alkali metal periodates, alkali metal hypobromites, alkali metal hypochlorites, alkali metal hypoiodites, alkali metal peroxides, and hydrogen peroxide.

6. The process of claim 5 wherein the depolymerizing agent is potassium chlorate or sodium chlorate.

7. The process of any one of claims 1 to 6 wherein the cellulose ether is alkyl cellulose, hydroxyalkylcellulose or hydroxyalkyl alkylcellulose.

8. The process of any one of claims 1 to 7 wherein the aliphatic monocarboxylic acid anhydride is selected from the group consisting of acetic anhydride, butyric anhydride and propionic anhydride and the dicarboxylic acid anhydride is selected from the group consisting of succinic anhydride, maleic anhydride and phthalic anhydride.

9. The process of any one of claims 1 to 8 wherein hydroxypropyl methylcellulose is esterified with succinic anhydride and acetic anhydride to produce hydroxypropyl methyl cellulose acetate succinate.

10. The process of any one of claims 1 to 9 wherein the weight average molecular weight of the esterified cellulose ether is at least 25 percent lower than the weight average molecular weight of the cellulose ether used in the esterification step as a starting material.

11. The process of any one of claims 1 to 10 wherein the molar ratio [dicarboxylic acid anhydride / anhydroglucose units of the cellulose ether] is from 0.2 / 1 to 1.0/1.

12. The process of any one of claims 1 to 11, wherein the amount of depolymerizing agent is from 0.03 to 1 mole per mole of anhydroglucose units of the cellulose ether.

13. The process of any one of claims 1 to 12 wherein the esterification reaction is conducted in the presence of from 200 to 1500 weight parts of an aliphatic carboxylic acid per 100 weight parts of cellulose ether.

14. The process of any one of claims 1 to 13 wherein the esterification reaction is conducted in the presence of an alkali metal carboxylate.

15. The process of any one of claims 1 to 14 wherein the cellulose ether has a viscosity of from 1000 mPa·s to 7,000 mPa·s, measured as a 2 weight-% solution in water at 20 °C, the amount of depolymerizing agent is from 0.03 to 0.30 mole per mole of anhydroglucose units of the cellulose ether, and the depolymerizing agent is an oxidizing agent different from oxygen.

**Patentansprüche**

1.  Verfahren zur Herstellung eines veresterten Celluloseethers, umfassend die Schritte des Umsetzens eines Celluloseethers mit einem aliphatischen Monocarbonsäureanhydrid und/oder einem Dicarbonsäureanhydrid in Gegenwart von bis zu 2.000 Gewichtsteilen einer aliphatischen Carbonsäure pro 100 Gewichtsteile Celluloseether und mit einem Depolymerisierungsmittel, wobei der Celluloseether eine anfängliche Viskosität von mindestens 500 mPa·s, gemessen als eine 2-gew.-%ige Lösung in Wasser bei 20°C, aufweist, das Depolymerisierungsmittel eine anorganische Säure oder ein Oxidationsmittel, das sich von Sauerstoff unterscheidet, ist und die Reaktion des Celluloseethers mit dem aliphatischen Monocarbonsäureanhydrid und/oder dem Dicarbonsäureanhydrid und mit dem Depolymerisierungsmittel gleichzeitig oder nacheinander in einer Reaktionsvorrichtung durchgeführt wird.

2.  Verfahren nach Anspruch 1, wobei der Celluloseether eine Viskosität von mindestens 1.000 mPa·s, gemessen als eine 2-gew.-%ige Lösung in Wasser bei 20°C, aufweist.

3.  Verfahren nach Anspruch 1 oder 2, wobei die Reaktionsmischung, die den Celluloseether, das aliphatische Monocarbonsäureanhydrid und/oder das Dicarbonsäureanhydrid und das Depolymerisierungsmittel enthält, hergestellt wird und die Reaktionsmischung danach erwärmt wird, um die Veresterungsreaktion durchzuführen.

4.  Verfahren nach einem der Ansprüche 1 bis 3, wobei die Menge des Depolymerisierungsmittels mindestens 0,03 mol pro mol Anhydroglucoseeinheit des Celluloseethers beträgt.

5.  Verfahren nach einem der Ansprüche 1 bis 4, wobei das Depolymerisierungsmittel ausgewählt ist aus der Gruppe bestehend aus Alkalimetallchloriten, Alkalimetallchloraten, Alkalimetallperchloraten, Alkalimetallperiodaten, Alkalimetallhypobromiten, Alkalimetallhypochloriten, Alkalimetallhypoioditen, Alkalimetallperoxiden und Wasserstoffperoxid.

6.  Verfahren nach Anspruch 5, wobei das Depolymerisierungsmittel Kaliumchlorat oder Natriumchlorat ist.

7.  Verfahren nach einem der Ansprüche 1 bis 6, wobei der Celluloseether Alkylcellulose, Hydroxyalkylcellulose oder Hydroxyalkylalkylcellulose ist.

8.  Verfahren nach einem der Ansprüche 1 bis 7, wobei das aliphatische Monocarbonsäureanhydrid ausgewählt ist aus der Gruppe bestehend aus Essigsäureanhydrid, Buttersäureanhydrid und Propionsäureanhydrid und das Dicarbonsäureanhydrid ausgewählt ist aus der Gruppe bestehend aus Bernsteinsäureanhydrid, Maleinsäureanhydrid und Phthalsäureanhydrid.

9.  Verfahren nach einem der Ansprüche 1 bis 8, wobei Hydroxypropylmethylcellulose mit Bernsteinsäureanhydrid und Essigsäureanhydrid verestert wird, um Hydroxypropylmethylcelluloseacetatsuccinat herzustellen.

10. Verfahren nach einem der Ansprüche 1 bis 9, wobei das gewichtsmittlere Molekulargewicht des veresterten Celluloseethers mindestens 25 % niedriger als das gewichtsmittlere Molekulargewicht des Celluloseethers ist, der in dem Veresterungsschritt als Ausgangsmaterial eingesetzt wird.

11. Verfahren nach einem der Ansprüche 1 bis 10, wobei das Molverhältnis [Dicarbonsäureanhydrid / Anhydroglucoseeinheiten des Celluloseethers] von 0,2/1 bis 1,0/1 beträgt.

12. Verfahren nach einem der Ansprüche 1 bis 11, wobei die Menge des Depolymerisierungsmittels von 0,03 bis 1 mol pro mol Anhydroglucoseeinheiten des Celluloseethers reicht.

13. Verfahren nach einem der Ansprüche 1 bis 12, wobei die Veresterungsreaktion in Gegenwart von 200 bis 1.500 Gewichtsteilen einer aliphatischen Carbonsäure pro 100 Gewichtsteile Celluloseether durchgeführt wird.

14. Verfahren nach einem der Ansprüche 1 bis 13, wobei die Veresterungsreaktion in Gegenwart eines Alkalimetallcarboxylats durchgeführt wird.

15. Verfahren nach einem der Ansprüche 1 bis 14, wobei der Celluloseether eine Viskosität von 1.000 mPa.s bis 7.000 mPa.s, gemessen als eine 2-gew.-%ige Lösung in Wasser bei 20°C, aufweist, die Menge des Depolymerisierungsmittels von 0,03 bis 0,30 mol pro mol Anhydroglucoseeinheiten des Celluloseethers reicht und das Depolymerisierungsmittel ein Oxidationsmittel ist, das sich von Sauerstoff unterscheidet.

**Revendications**

1. Un procédé pour produire un éther de cellulose estérifié comprenant l'étape consistant à faire réagir un éther de cellulose avec un anhydride d'acide monocarboxylique aliphatique et/ou un anhydride d'acide dicarboxylique en présence de jusqu'à 2 000 parties en poids d'un acide carboxylique aliphatique pour 100 parties en poids d'éther de cellulose et avec un agent de dépolymérisation, dans lequel l'éther de cellulose a une viscosité initiale d'au moins 500 mPa•s, mesurée en tant que solution à 2 % en poids dans de l'eau à 20 °C, l'agent de dépolymérisation est un acide inorganique ou un agent d'oxydation différent de l'oxygène et la réaction de l'éther de cellulose avec l'anhydride d'acide monocarboxylique aliphatique et/ou l'anhydride d'acide dicarboxylique et avec l'agent de dépolymérisation est effectuée simultanément ou en séquence dans un dispositif de réaction.

2. Le procédé de la revendication 1 dans lequel l'éther de cellulose a une viscosité d'au moins 1 000 mPa•s, mesurée en tant que solution à 2 % en poids dans de l'eau à 20 °C.

3. Le procédé de la revendication 1 ou de la revendication 2 dans lequel un mélange réactionnel comprenant l'éther de cellulose, l'anhydride d'acide monocarboxylique aliphatique et/ou l'anhydride d'acide dicarboxylique et l'agent de dépolymérisation est préparé et le mélange réactionnel est subséquemment chauffé afin d'effectuer la réaction d'estérification.

4. Le procédé de n'importe laquelle des revendications 1 à 3 dans lequel la quantité de l'agent de dépolymérisation est d'au moins 0,03 mole par mole d'unités anhydroglucose de l'éther de cellulose.

5. Le procédé de n'importe laquelle des revendications 1 à 4 dans lequel l'agent de dépolymérisation est sélectionné dans le groupe constitué de chlorites de métal alcalin, de chlorates de métal alcalin, de perchlorates de métal alcalin, de periodates de métal alcalin, d'hypobromites de métal alcalin, d'hypochlorites de métal alcalin, d'hypoiodites de métal alcalin, de peroxydes de métal alcalin, et de peroxyde d'hydrogène.

6. Le procédé de la revendication 5 dans lequel l'agent de dépolymérisation est du chlorate de potassium ou du chlorate de sodium.

7. Le procédé de n'importe laquelle des revendications 1 à 6 dans lequel l'éther de cellulose est l'alkyl cellulose, l'hydroxyalkylcellulose ou l'hydroxyalkyl alkylcellulose.

8. Le procédé de n'importe laquelle des revendications 1 à 7 dans lequel l'anhydride d'acide monocarboxylique aliphatique est sélectionné dans le groupe constitué d'anhydride acétique, d'anhydride butyrique et d'anhydride propionique et l'anhydride d'acide dicarboxylique est sélectionné dans le groupe constitué d'anhydride succinique, d'anhydride maléique et d'anhydride phtalique.

9. Le procédé de n'importe laquelle des revendications 1 à 8 dans lequel l'hydroxypropyl méthylcellulose est estérifiée avec de l'anhydride succinique et de l'anhydride acétique pour produire du succinate acétate d'hydroxypropyl méthyl cellulose.

10. Le procédé de n'importe laquelle des revendications 1 à 9 dans lequel la masse moléculaire moyenne en poids de l'éther de cellulose estérifié est d'au moins 25 pour cent plus faible que la masse moléculaire moyenne en poids de l'éther de cellulose utilisé dans l'étape d'estérification comme matière de départ.

11. Le procédé de n'importe laquelle des revendications 1 à 10 dans lequel le rapport molaire [anhydride d'acide dicarboxylique/unités anhydroglucose de l'éther de cellulose] va de 0,2/1 à 1,0/1.

12. Le procédé de n'importe laquelle des revendications 1 à 11, dans lequel la quantité d'agent de dépolymérisation va de 0,03 à 1 mole par mole d'unités anhydroglucose de l'éther de cellulose.

13. Le procédé de n'importe laquelle des revendications 1 à 12 dans lequel la réaction d'estérification est effectuée en présence de 200 à 1 500 parties en poids d'un acide carboxylique aliphatique pour 100 parties en poids d'éther de cellulose.

**14.** Le procédé de n'importe laquelle des revendications 1 à 13 dans lequel la réaction d'estérification est effectuée en présence d'un carboxylate de métal alcalin.

**15.** Le procédé de n'importe laquelle des revendications 1 à 14 dans lequel l'éther de cellulose a une viscosité allant de 1 000 mPa•s à 7 000 mPa•s, mesurée en tant que solution à 2 % en poids dans de l'eau à 20 °C, la quantité d'agent de dépolymérisation va de 0,03 à 0,30 mole par mole d'unités anhydroglucose de l'éther de cellulose, et l'agent de dépolymérisation est un agent d'oxydation différent de l'oxygène.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4226981 A
- US 5776501 A
- WO 2005115330 A
- WO 2011159626 A
- EP 1141029 A
- EP 210917 A
- EP 1423433 A
- US 4316982 A
- US 3629237 A
- EP 0219426 A
- US 13030394 W
- WO 2013148154 A

### Non-patent literature cited in the description

- **KEARY, C.M.** *Carbohydrate Polymers,* 2001, vol. 45, 293-303
- Hypromellose. USP, vol. 35, 423-424, 3467-3469
- **G. BARTELMUS ; R. KETTERER.** *Z. Anal. Chem.,* 1977, vol. 286, 161-190
- Hypromellose. European Pharmacopeia Ph Eur, 2008, vol. 6, 2819
- *Hypromellose,* 423-424, 3467-3469
- Hypromellose Acetate Succinate. United States Pharmacopia and National Formulary, vol. 29, 1548-1550
- Hypromellose. United States Pharmacopeia and National Formulary, vol. 35, 3467-3469
- *Journal of Pharmaceutical and Biomedical Analysis,* 2011, vol. 56, 743-748
- Hypromellose. USP, vol. 35, 423-424, 3467-3469
- Hypromellose. United States Pharmacopeia and National Formulary, USP, vol. 35, 3467-3469
- *Journal of Pharmaceutical and Biomedical Analysis,* 2011, vol. 56, 743
- **CHEN, R. et al.** *Journal of Pharmaceutical and Biomedical Analysis,* 2011, vol. 56, 743-748